# EUROPÄISCHE PATENTANMELDUNG

(11) **EP 4 574 810 A1**
(43) Veröffentlichungstag der Anmeldung: **25.06.2025**
(21) Anmeldenummer: 24218171.7
(22) Anmeldetag: 06.12.2024
(51) Int. Cl.: C07C 67/303, C07C 69/75, C07C 69/753, C07C 69/74, B01J 8/10

(54) **STATISCHE MISCHER IN DER HERSTELLUNG VON ALICYCLISCHEN CARBONSÄUREN UND DEREN ESTERN**

(30) Priorität: 18.12.2023 EP 23217548
(71) Anmelder: Evonik Oxeno GmbH & Co. KG, 45772 Marl (DE)
(72) Erfinder: Ziomek, Grzegorz, 45665 Recklinghausen (DE); Schneider, Thomas, 46514 Schermbeck (DE); Grass, Michael, 45721 Haltern am See (DE); Simon, Berno, 45772 Marl (DE)
(74) Vertreter: Evonik Patent Association

(57) **Zusammenfassung**

Die Erfindung liegt auf dem Gebiet der Herstellung von alicyclischen Verbindungen durch Kernhydrierung von aromatischen Verbindungen. Es werden im Rahmen der Erfindung Verfahren zur Herstellung alicyclischer Verbindungen, vorzugsweise alicyclischer Carbonsäuren und deren Estern, unter Verwendung eines statischen Mischers bereitgestellt sowie apparative Vorrichtungen zur Durchführung dieses Verfahrens.

## Beschreibung

Die Erfindung liegt auf dem Gebiet der Herstellung von alicyclischen Verbindungen durch Kernhydrierung von aromatischen Verbindungen. Es werden im Rahmen der Erfindung Verfahren zur Herstellung alicyclischer Verbindungen, vorzugsweise alicyclischer Carbonsäuren und deren Estern, unter Verwendung eines statischen Mischers bereitgestellt sowie apparative Vorrichtungen zur Durchführung dieses Verfahrens.

Alicyclische Polycarbonsäureester, wie beispielsweise die Ester der Cyclohexan-1 ,2-dicarbonsäure, werden als Schmierölkomponente und als Hilfsmittel bei der Metallverarbeitung eingesetzt. Weiterhin finden sie als Weichmacher für Polyolefine und für PVC Verwendung.

Für die Weichmachung von PVC werden überwiegend Ester der Phthalsäure, wie beispielweise Dinonyl- oder Didecylester, verwendet. Die Verwendung dieser Phthalate wird in der Öffentlichkeit zunehmend kontrovers diskutiert und deren Verwendung in Kunststoffen könnte limitiert werden. Alicyclische Polycarbonsäureester, von denen einige in der Literatur bereits als Weichmacher für Kunststoffe beschrieben sind, können eine geeignete Wahl für mögliche Ersatzstoffe der limitierten Weichmacher darstellen.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:
US 3 027 398 offenbart die Hydrierung von Dimethylterephthalat an geträgerten Ruthenium-Katalysatoren bei 110 bis 140°C und 35 bis 105 bar.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250 °C und 30 bis 200 bar hydriert.

In WO 99/32427 und WO 00/78704 werden Verfahren zur Hydrierung von Benzolpolycarbonsäureestern zu den entsprechenden alicyclischen Verbindungen offengelegt.

Die Hydrierung von aromatischen Verbindungen findet zum Beispiel durch Reaktion eines Aromatenhaltigen Edukts in der Flüssigphase mit einem wasserstoffhaltigen Hydriergas statt. Hierfür muss das Hydriergas in der Flüssigphase gelöst sein.

Bei Einleitung des Hydriergases und des flüssigen Eduktes in eine Hydriereinheit kommt es über die Länge der Hydriereinheit zu einer Konzentrationszunahme von Wasserstoff, der von der Gasphase in der Flüssigphase übergegangen ist. Dies führt zu unterschiedlichen Reaktionsgeschwindigkeiten innerhalb der Hydriereinheit und demnach unterschiedlichen Raum-Zeit-Ausbeuten in der Hydriereinheit. Im Gesamtkontext führt dieses Konzentrationsgefälle daher zu einer verminderten Gesamtausbeute eines Hydrierverfahrens.

Die primäre Aufgabe der vorliegenden Erfindung war es daher, ein Verfahren zur Herstellung von alicyclischen Verbindungen, vorzugsweise von alicyclischen Carbonsäuren und deren Estern bereitzustellen, welches eine annähernd konstante Raum-Zeit Ausbeute über die gesamte Länge der Hydriereinheit ermöglicht.

Diese primäre Aufgabe der vorliegenden Erfindung wurde dahingehend gelöst, dass ein Verfahren zur Herstellung einer oder mehrerer alicyclischer Verbindungen bereitgestellt wird, umfassend die Schritte:
i. Bereitstellen eines Stroms A aus einer oder mehrerer aromatischer Verbindungen sowie eines Stroms B aus einem wasserstoffhaltigen Hydriergas;
ii. In Kontaktbringen der in Schritt i. bereitgestellten Ströme A und B mittels mindestens eines bis maximal 8 statischen Mischern (4) unter Erhalt eines vermischten Stroms C (5) und Einbringen von Strom C (5) in eine Hydriereinheit (6);
iii. Hydrieren der einen oder mehreren aromatischen Verbindungen zu einer oder mehreren entsprechenden alicyclischen Verbindungen in der Hydriereinheit (6);
iv. Erhalten einer Produktmischung umfassend eine oder mehrere alicyclische Verbindungen.

Ein statischer Mischer ist ein Mischelement, welches durch seine interne Geometrie in der Lage ist, zwei oder mehrere Eingangsströme miteinander zu vermischen. Ein statischer Mischer umfasst ein Rohr, das Stromelemente bzw. Einbauten aufweist. Die Vermischung geschieht dadurch, dass die Eingangsströme über die Stromelemente im Rohr geführt werden und sich dadurch turbulente Strömungen ausbilden können. Statische Mischer sind in der Lage, Stoffsysteme aus zwei oder mehreren Komponenten mit den Phasen gasförmig, flüssig, überkritisch oder partikulär zu vermischen.

Im vorliegenden Fall wird mindestens ein statischer Mischer eingesetzt. Es können auch bis zu 8 statische Mischer eingesetzt werden, die parallel oder in Reihe geschaltet vorliegen. Die Verwendung einer möglichst geringen Anzahl an statischen Mischern ist schon aus Kostengründen vorteilhaft. Ein statischer Mischer umfasst ein Rohr, in das die beiden Ströme A und B über einen gemeinsamen Einlass zugeführt werden. In dem Rohr ist mindestens ein und sind vorzugsweise mehrere Stromelemente angeordnet. Durch das/die Stromelement(e) wird die gewünschte Vermischung der beiden Ströme A und B erreicht und es entsteht Strom C.

Im Rahmen dieser Erfindung wurde gefunden, dass der Einbau eines statischen Mischers in eine Hydriereinheit dazu führt, dass eine Vermischung der Edukte vor Eintritt in die Hydriereinheit erfolgt und so die Konzentration an Wasserstoff in der flüssigen Phase gleich zu Anfang stark erhöht ist. Dies führt zu einer verbesserten Reaktion im Anfangsbereich der Hydriereinheit.

Unter "alicyclischen Verbindungen" im Rahmen der vorliegenden Erfindung sind solche Verbindungen zu verstehen, die ein gesättigtes Ringsystem mit einer aliphatischen Struktur besitzen. Solche Verbindung sind auch als cycloaliphatische Verbindungen bekannt. Vorzugsweise besitzen die alicyclischen Verbindungen, die im Rahmen der vorliegenden Erfindung als Produkte erhalten werden, einen Cylohexanring.

Unter "aromatischen Verbindungen" im Rahmen der vorliegenden Erfindung sind solche Verbindungen zu verstehen, die mindestens ein Ringsystem besitzen, das nach der Hückel-Regel in konjugierten Doppelbindungen, freien Elektronenpaaren oder unbesetzten p-Orbitalen eine Anzahl von 4n+2 delokalisierten Elektronen enthält. Vorzugsweise besitzen die aromatischen Verbindungen, die im Rahmen der vorliegenden Erfindung als Edukte eingesetzt werden, einen Benzolring.

Ein "wasserstoffhaltiges Hydriergas" ist ein Gas, welches Wasserstoff enthält. Wasserstoff wird im Rahmen der der vorliegenden Erfindung zu Grunde liegenden Reaktion neben den aromatischen Verbindungen als weiteres Edukt gebraucht. Bei der durchgeführten Hydrierreaktion werden die Doppelbindungen im Ring der eingesetzten aromatischen Verbindungen, vorzugsweise des Benzolrings, durch eine Additionsreaktion des Wasserstoffs hydriert und dadurch aufgelöst. Die Reaktion findet in Anwesenheit eines Feststoffkatalysators statt. Hierbei bindet das Wasserstoffmolekül auf dem wasserstoffhaltigen Hydriergas intermediär an das Metallatom des Katalysators und die Bindung zwischen den beiden Wasserstoffatomen im Wasserstoffmolekül wird geschwächt und kann mit einer elektronenreichen Mehrfachbindung (Doppelbindung) wechselwirken. Die Hydrierung findet statt, wenn formal jeweils zwei Wasserstoffatome auf eine Doppelbindung übertragen werden. Damit werden die Doppelbindungen in den aromatischen Verbindungen aufgelöst und es werden alicyclische Verbindungen erhalten.

Als Hydriergase können beliebige wasserstoffhaltige Gasgemische, die keine schädlichen Mengen an Katalysatorgiften, wie beispielsweise Kohlenmonoxid oder Schwefelwasserstoff enthalten, eingesetzt werden. Die Verwendung von Inertgasen ist optional, bevorzugt wird Wasserstoff in einer Reinheit größer 95 %, insbesondere größer 98 % eingesetzt. Inertgasanteile können beispielsweise Stickstoff oder Methan sein. Vorzugsweise ist in den Hydriereinheiten so viel Wasserstoff vorhanden, dass dieser im Überschuss, insbesondere in einem Überschuss von 1 bis 200 %, bevorzugt in einem Überschuss von 3 bis 100 % und besonders bevorzugt in einem Überschuss von 5 bis 50 % bezogen auf die stöchiometrische Menge, die zur Erzielung des in der Hydriereinheit möglichen bzw. gewünschten Umsatzes benötigt wird, vorliegt. Das Einstellen eines ausreichenden Überschusses an Wasserstoff kann sich vorteilhaft auf die vollständige Hydrierung der aromatischen Bindungen auswirken.

Eine "Hydriereinheit" im Rahmen der vorliegenden Erfindung ist zu verstehen als Hydrierreaktor oder mehrere hintereinandergeschalteter Reaktoren oder mehrere parallel zueinander geschalteten Reaktoren oder eine Reaktorgruppe, die aus parallel und hintereinander geschalteten Reaktoren besteht. Daher zu verstehen als ein Reaktor oder eine Reaktoranordnung, die im erfindungsgemäßen Verfahren die Funktion eines Reaktors ausüben kann.

Die einzelnen Hydriereinheiten können mit Frisch-Wasserstoff beschickt werden. Um den Wasserstoffverbrauch und die mit dem Abgas bedingten Austragsverluste zu minimieren, ist es jedoch zweckmäßig, das Abgas einer Hydriereinheit als Hydriergas einer anderen oder der gleichen Hydriereinheit zu verwenden. Weiterhin kann das Abgas einer Hydriereinheit nach Aufarbeitung wieder als Frisch-Wasserstoff eingesetzt werden. Beispielsweise ist es bei einem Verfahren, das in zwei hintereinander geschalteten Hydriereinheiten mit jeweils einem Reaktor durchgeführt wird, vorteilhaft, Frisch-Wasserstoff in die erste Hydriereinheit einzuspeisen und das Abgas der ersten Hydriereinheit in die zweite Hydriereinheit zu leiten. In diesem Falle können Edukt und Hydriergas in entgegengesetzter Reihenfolge durch die Hydriereinheiten strömen oder beispielsweise mittels eines statischen Mischers vorher vermischt werden. Es ist bei dieser Verfahrensführung vorteilhaft, den Wasserstoffüberschuss, bezogen auf die stöchiometrisch notwendige Menge, unter 30 %, insbesondere unter 20 % zu halten.

Die erfindungsgemäße Hydrierung wird vorzugsweise in der Flüssig/Gas-Mischphase oder Flüssigphase in zwei hintereinandergeschalteten Hydriereinheiten durchgeführt. Dabei wird die erste Hydriereinheit in Schlaufenfahrweise betrieben, d. h. ein Teil des Hydrieraustrags der ersten Hydriereinheit wird zusammen mit Frisch-Edukt auf den Kopf der ersten Hydriereinheit geleitet. Vorzugsweise erfolgt die erneute Zuleitung durch Einleiten in den statischen Mischer oder durch eine Zuleitung ohne einen statischen Mischer. Der andere Teil des Austrags der ersten Hydriereinheit wird in einer zweiten Hydriereinheit im geraden Durchgang hydriert. Vorzugsweise kann in der Zuleitung zu der zweiten Hydriereinheit ebenfalls ein statischer Mischer vorhanden sein. Es ist auch möglich, anstelle einer großen Hydriereinheit in Schlaufenfahrweise mehrere kleinere Hydriereinheiten in Schlaufenfahrweise, die in Reihe oder parallel angeordnet sind, zu verwenden. Ebenso ist es möglich, anstatt einer großen Hydriereinheit, die im geraden Durchgang durchströmt wird, mehrere Hydriereinheiten, die in Reihe oder parallel miteinander verschaltet sind, zu betreiben. Vorzugsweise ist in jeder Zuleitung zu den einzelnen Hydriereinheiten ein statischer Mischer vorhanden. Bevorzugt werden eine Hydriereinheit, die in Schlaufenfahrweise betrieben wird, und eine Hydriereinheit, die im geraden Durchgang betrieben wird, verwendet.

Die Hydrierung kann in Ab- oder vorzugsweise in Anwesenheit eines Lösungsmittels durchgeführt werden. Als Lösungsmittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten. Vorzugsweise, bei Anwesenheit eines Lösungsmittels, wird dieses ebenfalls mittels des statischen Mischers mit den in Schritt i. bereitgestellten Edukten vermischt.

Beispielsweise können folgende Stoffe als Lösungsmittel eingesetzt werden: Geradkettige oder cyclische Ether, wie beispielsweise Tetrahydrofuran oder Dioxan, sowie aliphatische Alkohole, in denen der Alkylrest 1 bis 13 Kohlenstoffatome aufweist.

Bevorzugt verwendbare Alkohole als Lösungsmittel sind Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösungsmittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Zulauf zu der Hydriereinheit begrenzt werden, wodurch eine bessere Temperaturkontrolle in der Hydriereinheit erreicht werden kann. Dies kann eine Minimierung von Nebenreaktionen und somit eine Erhöhung der Produktausbeute zur Folge haben. Bevorzugt liegt der Gehalt an aromatischen Verbindungen im Zulauf zu der Hydriereinheit zwischen 1 und 35 Gew.-%, vorzugsweise zwischen 2 und 25 Gew.-% bezogen auf die Gesamteduktmenge. Der gewünschte Konzentrationsbereich kann bei Hydriereinheiten, die in Schlaufenfahrweise betrieben werden, durch das Kreislaufverhältnis (Mengenverhältnis von rückgeführten Hydrieraustrag zu Edukt) eingestellt werden.

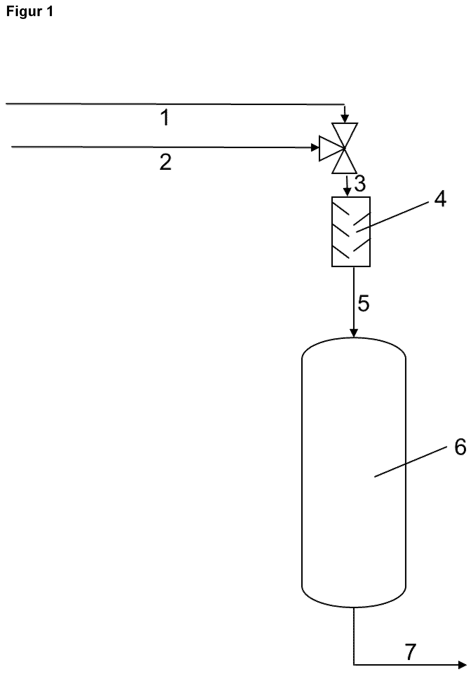

Nachfolgend wird das erfindungsgemäße Verfahren am Beispiel des in Figur 1 gezeigten Aufbaus beispielsweise beschrieben:
Die Edukte werden über mindestens zwei Rohrleitungen (1, 2), die gegebenenfalls zu einem einzigen Strom (3) kombiniert werden, zu dem statischen Mischer zugeführt. Vorzugsweise befindet sich in jeweils einer der beiden Rohrleitungen ein wasserstoffhaltiges Hydriergas und aromatische Verbindungen in flüssiger Form. Diese werden anschließend in den statischen Mischer (4) eingeleitet und über die Rohrleitung (5) als homogenes Gemisch in die Hydriereinheit (6) zugeführt, in welcher die Hydrierreaktion stattfindet. Der Produktstrom (7) enthält ein Gemisch aus alicyclischen Verbindungen als Produkt und eine verbleibende Restkonzentration des Edukts.

Vorzugsweise enthält der Produktstrom (7) am Ausgang der Hydriereinheit weniger als 0,3 Gew.-%, vorzugsweise weniger als 0,1 Gew.-%, insbesondere weniger als 0,05 Massen-%, besonders bevorzugt 0,005 Massen-% als Edukt eingesetzter aromatischer Verbindungen.

Es ist bevorzugt, dass der statische Mischer eine Geometrie aufweist, welche eine Strömung mit einer Reynoldszahl von größer als 100, vorzugsweise von größer als 200, insbesondere von größer als 500, besonders bevorzugt von größer als 900 im statischen Mischer vermittelt.

Der Fachmann ist in der Lage die Reynoldszahl basierend auf der Geometrie eines statischen Mischers zu bestimmen. Die allgemeine Formel der Reynoldszahl ist dem Fachmann bekannt.

Es ist weiterhin bevorzugt, dass der statische Mischer eine Geometrie aufweist, welche eine Vermischung des in Schritt i. bereitgestellten Hydriergases und der bereitgestellten aromatischen Verbindungen in einer flüssigen Phase derart vermittelt, dass der Wasserstoff aus dem wasserstoffhaltigen Hydriergas in der flüssigen Phase am Ende des statischen Mischers annähernd in Sättigungskonzentration vorliegt. Der Fachmann ist in der Lage die Sättigungskonzentration in einer flüssigen Phase zu bestimmen.

Unter "annähernd in Sättigungskonzentration" im Rahmen der vorliegenden Erfindung ist gemeint, dass die Konzentration des Wasserstoffs in der flüssigen Phase maximal 15 %, vorzugsweise maximal 10 %, besonders bevorzugt maximal 5 % und ganz besonders bevorzugt maximal 1 % unterhalb der Sättigungskonzentration liegt. Das bedeutet beispielsweise, dass wenn die Sättigungskonzentration des Wasserstoffs in der flüssigen Phase 1 g/L beträgt, dann bedeutet eine Konzentration von 0,85 g/L Wasserstoff in der flüssigen Phase ebenfalls, dass eine Sättigungskonzentration vorliegt.

Daher ist es weiterhin bevorzugt, dass der statische Mischer eine Bauweise ausgewählt aus der Gruppe bestehend aus den Mischertypen Kenics-Mischer, Sulzer SMV-Mischer, Sulzer SMX-Mischer, Fluitec CSE-Mischer und Ross-ISG-Mischer, vorzugsweise eine Kenics-Mischer Bauweise, besitzt.

Ein Kenics-Mischer weist eine Geometrie auf, die den Eingangsstrom durch ein spiralförmiges Mischelement radial in Richtung der Rohrwände und zurück zur Mitte leitet. Eine zusätzliche Umkehrung der Fließrichtung und eine Stromaufteilung ergeben sich aus der Kombination von abwechselnd nach links und rechts gedrehten Elementen. Durch diese Fließprofile werden die Eingangsströme gemischt.

Es ist bevorzugt im Zusammenhang mit der vorliegenden Erfindung, dass das Hydrieren der in Schritt i. bereitgestellten aromatischen Verbindungen an einem oder mehreren in einem Festbett der Hydriereinheiten angeordneten Feststoff-Katalysatoren mit dem in Schritt i. bereitgestellten wasserstoffhaltigen Gas erfolgt.

Es ist weiterhin bevorzugt, dass der Feststoff-Katalysator mindestens ein Metall der achten Nebengruppe des Periodensystems der Elemente aufweist. Vorzugsweise werden als Aktivmetalle Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird.

Neben den bereits genannten Metallen wird vorzugsweise zusätzlich mindestens ein Metall der ersten und/oder siebten Nebengruppe des Periodensystems der Elemente in den Katalysatoren enthalten sein. Bevorzugt wird Rhenium und/oder Kupfer zusätzlich zu dem Metall der achten Nebengruppe des Periodensystems der Elemente eingesetzt.

Die im Rahmen dieses Verfahren eingesetzte Katalysatoren sind auf ein Trägermaterial aufgebrachte Metalle wie vorhergehend definiert. Vorzugsweise handelt es sich bei den eingesetzten Trägermaterialien um Materialien enthaltend Mikroporen (Porendurchmesser kleiner 2 nm), Mesoporen (Porendurchmesser 2 bis 50 nm) und Makroporen (Porendurchmesser größer 50 nm). So sind hinsichtlich der Porenart Trägermaterialien mit folgenden Porenkombinationen einsetzbar:
a) nur Mesoporen,
b) Mikroporen und Mesoporen,
c) Mesoporen und Makroporen,
d) Mikroporen und Mesoporen und Makroporen,
e) Mikroporen und Makroporen.

Vorzugsweise werden als Trägermaterialien Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische eingesetzt.

Bevorzugt werden als Trägermaterialien Feststoffe eingesetzt, die unter Hydrierbedingungen weitgehend inert sind. Dies sind beispielsweise Aktivkohle, Siliciumcarbid, Siliciumdioxid, Titandioxid und/oder Zirkondioxid bzw. Gemische aus diesen Verbindungen. Ganz besonders bevorzugt werden Titandioxide als Trägermaterialien verwendet. Titandioxid tritt in drei Modifikationen auf (Anatas, Rutil, Brookit) auf, von denen Anatas und Rutil die häufigsten sind. Ein bevorzugtes Trägermaterial ist Aerolyst 7711^{®} (Evonik Operations GmbH). Dieses Trägermaterial besteht zu 15-20 Massen-% aus Rutil und zu 80-85 Massen-% aus Anatas. Weitere geeignete Titandioxidträgermaterialien sind beispielsweise solche, die auf Basis von Titanoxiden aus einem Schwefelsäure-Verfahren hergestellt werden. Sie enthalten in der Regel > 98 % Anatas.

Im erfindungsgemäßen Verfahren wird die Hydrierung in Schritt iii. in flüssiger Phase oder in der Gasphase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt-/Eduktphase hauptsächlich im flüssigen Zustand befindet, bevorzugt.

Es ist bevorzugt, dass das Hydrieren in Schritt iii. bei einem Druck von 3 bis 300 bar, vorzugsweise 15 bis 200 bar, besonders bevorzugt 50 bis 150 bar, durchgeführt wird.

Weiterhin ist es bevorzugt, dass das Hydrieren in Schritt iii. bei einer Temperatur von 50°C bis 250°C, vorzugsweise 70 bis 200°C, durchgeführt wird. Aufgrund der Exothermie der Hydrierreaktion erfolgt die Reaktion nicht bei einer festen Temperatur, sondern in einem Temperaturbereich wie hierin beschrieben. So nimmt die Temperatur des Reaktionsgemisches bei Durchfließen der Hydriereinheit zu.

Es ist im Rahmen des erfindungsgemäßen Verfahrens bevorzugt, dass in Schritt i. ein oder mehrere aromatische Carbonsäureester, vorzugsweise ein oder mehrere aromatische Mono-, Di- und Polycarbonsäureestern bereitgestellt werden.

Im Rahmen des erfindungsgemäßen Verfahrens können aromatische Verbindungen, wie aromatische Poly- und/oder Monoarbonsäuren oder deren Derivate, insbesondere deren Alkylester zu den entsprechenden alicyclischen Polycarbonsäureverbindungen umgesetzt werden. Dabei können sowohl Vollester als auch Partialester hydriert werden. Unter Vollester wird eine Verbindung verstanden, bei der alle Säuregruppen verestert sind. Partialester sind Verbindungen mit mindestens einer freien Säuregruppe (oder ggf. einer Anhydridgruppe) und mindestens einer Estergruppe.

Werden im erfindungsgemäßen Verfahren Polycarbonsäureester eingesetzt, so enthalten diese bevorzugt 2, 3 oder 4 Esterfunktionen.

Es ist im Rahmen des erfindungsgemäßen Verfahrens bevorzugt, dass in Schritt i. ein oder mehrere Benzol-, Diphenyl-, Naphthalin-, Diphenyloxid, Anthracen-Di- oder Polycarbonsäureester bereitgestellt werden. Die mit dem erfindungsgemäßen Verfahren erhaltenen alicyclischen Polycarbonsäuren bzw. deren Derivate bestehen aus einem oder mehreren, ggf. durch eine C-C-Bindung verknüpfte oder ankondensierte C₆-Ringe.

Weiterhin ist es bevorzugt, dass in Schritt i. ein oder mehrere aromatische Carbonsäureester mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise C₈-C₁₀-Phthalat, C₈-C₁₀-Terephthalat, C₈-C₁₀-Isophthalat und C₈-C₁₀-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Di-isononylisophthalat, Di-2-ethylhexyltrimellitat und Tri-isononyltrimellitat, bereitgestellt werden.

Hierbei bedeutet C₈ vorzugsweise 2-Ethylhexyl oder n-Octyl, C₉ bedeutet Isononyl und C₁₀ bedeutet Isodecyl oder 2-Propylheptyl.

Vorzugsweise ist das Verfahren ein Verfahren zur Hydrierung von 1,2-; 1,3- oder 1,4-Benzoldicarbonsäureester, und/oder der 1,2,3-; 1,2,4- oder 1,3,5-Benzoltricarbonsäureester, d. h. es werden die Isomere der 1,2-; 1,3- oder 1,4-Cyclohexandicarbonsäureester, oder der 1,2,3-; 1,3,5- oder 1,2,4-Cyclohexantricarbonsäureester erhalten.

Im erfindungsgemäßen Verfahren können beispielsweise Ester folgender aromatischer Carbonsäuren eingesetzt werden: 1,2-Naphthalindicarbonsäure, 1,3-Naphthalindicarbonsäure, 1,4-Naphthalindicarbonsäure, 1,5-Naphthalindicarbonsäure, 1,6-Naphthalindicarbonsäure, 1,7-Naphthalindicarbonsäure, 1,8-Naphthalindicarbonsäure, Phthalsäure (Benzol-1,2-dicarbonsäure), Isophthalsäure (Benzol-1,3-dicarbonsäure), Terephthalsäure (Benzol-1,4-dicarbonsäure), Benzol-1,2,3-tricarbonsäure, Benzol-1,2,4-tricarbonsäure (Trimellitsäure), Benzol-1,3,5-tricarbonsäure (Trimesinsäure), Benzol-1,2,3,4-tetracarbonsäure. Weiterhin können Säuren eingesetzt werden, die aus den genannten Säuren durch Substitution eines oder mehrerer am aromatischen Kern gebundenen Wasserstoffatome durch Alkyl-, Cycloalkyl- oder Alkoxyalkylgruppen entstehen.

Vorzugsweise werden Alkyl-, Cycloalkyl- sowie Alkoxyalkylester z. B. der oben genannten Säuren eingesetzt, wobei diese Reste unabhängig voneinander 1 bis 25, insbesondere 3 bis 15, ganz besonders 8 bis 13 C-Atome, insbesondere 9 C-Atome, umfassen. Diese Reste können linear oder verzweigt sein. Hat ein Edukt mehr als eine Estergruppe, dann können diese Reste gleich oder verschieden sein.

Im erfindungsgemäßen Verfahren können als Ester einer aromatischen Polycarbonsäure beispielsweise folgende Verbindungen eingesetzt werden: Terephthalsäuremonomethylester, Terephthalsäuredimethylester, Terephthalsäurediethylester, Terephthalsäuredi-n-propylester, Terephthalsäuredibutylester, Terephthalsäurediisobutylester, Terephthalsäuredi-tert.-butylester, Terephthalsäure-dipentylester, Terephthalsäuremonoglykolester, Terephthalsäurediglykolester, Terephthalsäure-n-octylester, Terephthalsäurediisooctylester, Terephthalsäuredi-2-ethylhexylester, Terephthalsäuredi-n-nonylester, Terephthalsäurediisononylester, Terephthalsäure-di-2-propylheptylester, Terephthalsäuredi-n-decylester, Terephthalsäuredi-n-undecylester, Terephthalsäurediisodecylester, Terephthalsäurediisododecylester, Terephthalsäure-ditridecylester, Terephthalsäuredi-n-octadecylester, Terephthalsäurediisooctadecylester, Terephthalsäuredi-n-eicosylester, Terephthalsäuremonocyclohexylester; Phthalsäuremonomethylester, Phthalsäuredimethylester, Phthalsäuredi-n-propylester, Phthalsäuredi-n-butylester, Phthalsäurediisobutylester, Phthalsäuredi-tert.-butylester, Phthalsäuremonoglykolester, Phthalsäurediglykolester, Phthalsäuredi-n-octylester, Phthalsäurediisooctylester, Phthalsäuredi-2-ethylhexylester, Phthalsäuredi-n-nonylester, Phthalsäurediisononylester, Phthalsäuredi-n-decylester, Phthalsäuredi-2-propylheptylester, Phthalsäurediisodecylester, Phthalsäuredi-n-undecylester, Phthalsäurediisoundecylester, Phthalsäureditridecylester, Phthalsäuredi-n-octadecylester, Phthalsäurediisooctadecylester, Phthalsäuredi-n-eicosylester, Phthalsäuremonocyclohexylester; Phthalsäuredicyclohexylester, Isophthalsäuremonomethylester, Isophthalsäuredimethylester, Isophthalsäurediethylester, Isophthalsäuredi-n-propylester, Isophthalsäuredi-n-butylester, Isophthalsäurediisobutylester, Isophthalsäuredi-tert.-butylester, Isophthalsäuremonoglykolester. Isophthalsäurediglykolester, Isophthalsäuredi-n-octylester, Isophthalsäurediisooctylester, Isophthalsäuredi-2-ethylhexylester, Isophthalsäuredi-n-nonylester, Isophthalsäurediisononylester, Isophthalsäuredi-n-decylester, Isophthalsäurediisodecylester, Isophthalsäuredi-n-undecylester, Isophthalsäurediisododecylester, Isophthalsäuredi-n-dodecylester, Isophthalsäureditridecylester, Isophthalsäuredi-n-octadecylester, Isophthalsäurediisooctadecylester, Isophthalsäuredi-n-eicosylester, Isophthalsäuremonocyclohexylester.

Das erfindungsgemäße Verfahren ist prinzipiell auch auf Benzoesäure und deren Ester anwendbar. Hierunter werden neben Benzoesäurealkylester auch Benzoate von Diolen, wie beispielsweise Glycoldibenzoat, Diethylenglycolbenzoat, Triethylenglycoldibenzoat oder Propylenglycoldibenzoat, verstanden. Die Alkoholkomponente der Benzoesäurealkylester kann aus 1 bis 25, bevorzugt 8 bis 13 Kohlenstoffatomen, jeweils linear oder verzweigt, bestehen.

Großtechnisch werden vorzugsweise aromatische Ester, insbesondere Vollester häufig aus Alkoholgemischen hergestellt. Entsprechende Alkoholgemische sind beispielsweise: C₅-Alkoholgemische, hergestellt aus linearen Butenen durch Hydroformylierung und anschließender Hydrierung; C₅-Alkoholgemische, hergestellt aus Butengemischen, die lineare Butene und Isobuten enthalten, durch Hydroformylierung und anschließende Hydrierung; C₆-Alkoholgemische, hergestellt aus einem Penten oder aus einem Gemisch von zwei oder mehreren Pentenen, durch Hydroformylierung und anschließende Hydrierung; C₇-Alkoholgemische, hergestellt aus der Trimerisierung von Ethylen oder Dimerisierung von Propylen oder einem Hexenisomer oder einem sonstigen Gemisch von Hexenisomeren, durch Hydroformylierung und anschließende Hydrierung; Ca-Alkoholgemische, wie 2-Ethylhexanol (2 Isomere), hergestellt durch Aldolkondensation von n-Butyraldehyd und anschließende Hydrierung; Cs-Alkoholgemische, hergestellt aus C₄-Olefinen durch Dimerisierung, Hydroformylierung und Hydrierung. Dabei kann zur Herstellung der Cs-Alkohole von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der Ca-Olefingemische kann mit Hilfe von Rhodium- oder Kobaltkatalysatoren erfolgen; C₁₀-Alkoholgemische, hergestellt aus Tripropylen durch Hydroformylierung und anschließende Hydrierung; 2-Propylheptanol (2 Isomere), hergestellt durch Aldolkondensation von Valeraldehyd und anschließende Hydrierung;
C₁₀-Alkoholgemische, hergestellt aus einem Gemisch von mindestens zwei C₅-Aldehyden durch Aldolkondensation und anschließende Hydrierung; C₁₃-Alkoholgemische, hergestellt aus Hexaethylen, Tetrapropylen oder Tributen durch Hydroformylierung und anschließende Hydrierung.

Weitere Alkoholgemische können durch Hydroformylierung und anschließende Hydrierung aus Olefinen bzw. Olefingemischen gewonnen werden, die beispielsweise bei Fischer-Tropsch-Synthesen, bei Dehydrierungen von Kohlenwasserstoffen, Metathesereaktionen, beim Polygasverfahren oder anderen technischen Prozessen anfallen.

Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Polycarbonsäuren und den oben genannten Alkoholgemischen, eingesetzt werden. Erfindungsgemäß werden bevorzugt Ester, hergestellt aus Phthalsäure oder Phthalsäureanhydrid und einem Gemisch isomerer Alkohole mit 4 bis 13 C-Atomen, eingesetzt.

Vorzugsweise umfasst das erfindungsgemäße Verfahren die Schritte:
i. Bereitstellen einer oder mehrerer aromatischer Verbindungen ausgewählt aus der Gruppe bestehend aus Estern der Phthalsäure, Isophthalsäure, Terephthalsäure und Trimellitsäure, besonders bevorzugt ausgewählt aus der Gruppe bestehend aus Terephthalsäuredi-n-butylester, Terephthalsäuredipentylester, Terephthalsäuredi-2-ethylhexylester, Terephthalsäurediisononylester, Phthalsäuredipentylester, Phthalsäuredi-2-ethylhexylester, Phthalsäurediisononylester, Isophthalsäuredipentylester, Isophthalsäuredi-2-ethylhexylester, Isophthalsäurediisononylester, Trimellitsäuretripentylester, Trimellitsäuretri-2-ethylhexylester, Trimellitsäuretriisononylester, oder Mischungen daraus,
   sowie eines wasserstoffhaltigen Hydriergases;
ii. In Kontaktbringen der in Schritt i. bereitgestellten Stoffe mittels eines statischen Mischers und Einbringen in eine Hydriereinheit (6);
iii. Hydrieren der einen oder mehreren aromatischen Verbindungen zu einer oder mehreren entsprechenden alicyclischen Verbindungen in der Hydriereinheit (6);
iv. Erhalten einer Produktmischung umfassend eine oder mehrere alicyclische Verbindungen ausgewählt aus der Gruppe bestehend aus 1,2-Dialkylcyclohexandicarbonsäureester, 1,3-Dialkylcyclohexandicarbonsäureester, 1,4-Dialkylcyclohexandicarbonsäureestern und 1,2,4-Cyclohexantricarbonsäureester, besonders bevorzugt ausgewählt aus der Gruppe bestehend aus 1,4-Cyclohexandicarbonsäuredi-n-butylester, 1,4-Cyclohexandicarbonsäuredipentylester, 1,4-Cyclohexandicarbonsäuredi2-ethylhexylester, 1,4-Cyclohexandicarbonsäurediisononylester, 1,2-Cyclohexandicarbonsäuredipentylester, 1,2-Cyclohexandicarbonsäuredi-2-ethylhexylester, 1,2-Cyclohexandicarbonsäurediisononylester, 1,3-Cyclohexandicarbonsäuredipentylester, 1,3-Cyclohexandicarbonsäuredi-2-ethylhexylester, 1,3-Cyclohexandicarbonsäurediisononylester, 1,2,4-Cyclohexantricarbonsäuretripentylester, 1,2,4-Cyclohexantricarbonsäuretri-2-ethylhexylester, 1,2,4-Cyclohexantricarbonsäuretriisononylester.

Das erhaltene Produkt ist abhängig von dem eingesetzten Edukt. So wird beispielsweise 1,2-Cyclohexandicarbonsäurediisononylester als Produkt enthalten, wenn Phthalsäurediisononylester als Edukt eingesetzt werden. 1,2-Cyclohexandicarbonsäuredi-2-ethylhexylester werden dementsprechend aus Phthalsäuredi-2-ethylhexylestern erhalten.

Vorzugsweise umfasst das erfindungsgemäße Verfahren die Schritte:
i. Bereitstellen eines Edukts enthaltend Phthalsäurediisononylester (DINP) oder Phthalsäuredi-2-ethylhexylester (DEHP) sowie eines wasserstoffhaltigen Hydriergases;
ii. In Kontaktbringen der in Schritt i. bereitgestellten Stoffe mittels eines statischen Mischers und Einbringen in eine Hydriereinheit (6);
iii. Hydrieren der einen oder mehreren aromatischen Verbindungen zu den entsprechenden alicyclischen Verbindungen in der Hydriereinheit (6);
iv. Erhalten einer Produktmischung umfassend 1,2-Cyclohexandicarbonsäurediisononylester oder 1,2-Cyclohexandicarbonsäuredi-2-ethylhexylester.

Zudem wird beispielsweise 1,4-Cyclohexandicarbonsäurediisononylester als Produkt enthalten, wenn Terephthalsäurediisononylester als Edukt eingesetzt werden. 1,4-Cyclohexandicarbonsäuredi-2-ethylhexylester werden dementsprechend aus Terephthalsäuredi-2-ethylhexylestern erhalten.

Vorzugsweise umfasst das erfindungsgemäße Verfahren die Schritte:
i. Bereitstellen eines Edukts enthaltend Terephthalsäurediisononylester oder Terephthalsäuredi-2-ethylhexylester sowie eines wasserstoffhaltigen Hydriergases;
ii. In Kontaktbringen der in Schritt i. bereitgestellten Stoffe mittels eines statischen Mischers und Einbringen in eine Hydriereinheit (6);
iii. Hydrieren der einen oder mehreren aromatischen Verbindungen zu den entsprechenden alicyclischen Verbindungen in der Hydriereinheit (6);
iv. Erhalten einer Produktmischung umfassend 1,4-Cyclohexandicarbonsäurediisononylester oder 1,4-Cyclohexandicarbonsäuredi-2-ethylhexylester.

Darüber hinaus wird beispielsweise 1,2,4-Cyclohexantricarbonsäuretriisononylester als Produkt enthalten, wenn Trimellitsäuretriisononylester als Edukt eingesetzt werden. 1,2,4-Cyclohexantricarbonsäuretri-2-ethylhexylester werden dementsprechend aus Trimellitsäuretri-2-ethylhexylestern erhalten.

Vorzugsweise umfasst das erfindungsgemäße Verfahren die Schritte:
i. Bereitstellen eines Edukts enthaltend Trimellitsäuretriisononylester (TINTM) oder Trimellitsäuretri-2-ethylhexylester (TOTM) sowie eines wasserstoffhaltigen Hydriergases;
ii. In Kontaktbringen der in Schritt i. bereitgestellten Stoffe mittels eines statischen Mischers und Einbringen in eine Hydriereinheit (6);
iii. Hydrieren der einen oder mehreren aromatischen Verbindungen zu den entsprechenden alicyclischen Verbindungen in der Hydriereinheit (6);
iv. Erhalten einer Produktmischung umfassend 1,2,4-Cyclohexantricarbonsäuretriisononylester oder 1,2,4-Cyclohexantricarbonsäuretri-2-ethylhexylester.

Das erfindungsgemäße Verfahren wird vorzugsweise unter folgenden Bedingungen durchgeführt:
Im Zulauf zu einer Hydriereinheit ist ein Kenics-Mischer verbaut, welche eine Reynoldszahl von über 100 im Mischer vermittelt. Im Mischerzulauf liegt die Konzentration der aromatischen Verbindungen als Edukt zwischen 5 und 30 Massen-%, insbesondere zwischen 8 und 15 Massen-%. Im Ausgangstrom der Hydriereinheit liegt die Konzentration des Edukts zwischen 0,3 und 8 Massen-%, insbesondere zwischen 1,5 und 4 Massen-%.

Nach Einleiten des Ausgangsstroms des statischen Mischers in die Hydriereinheit erfolgt die Hydrierreaktion an einem Katalysator.

Die spezifische Katalysatorbelastung (LHSV, Liter Frisch-Edukt je Liter Katalysator je Stunde) in der Hydriereinheit beträgt 0,1 bis 5, h⁻¹ insbesondere 0,5 bis 3 h⁻¹.

Die Oberflächenbelastung in der Hydriereinheit liegt im Bereich von 25 bis 140 m³/m²/h, insbesondere im Bereich von 50 bis 90 m³/m²/h.

Die durchschnittlichen Hydriertemperaturen in der Hydriereinheit sind 70 bis 150 °C, insbesondere 80 bis 120 °C.

Der Hydrierdruck in der Hydriereinheit beträgt 25 bis 200 bar, insbesondere 80 bis 110 bar.

Die Verfahrensvarianten eigenen sich insbesondere zur Hydrierung von Phthalsäureestern, besonders für Isononylphthalate (als Isomerengemisch "Diisononylphthalat" z. B. VESTINOL^{®}9 der Evonik OXENO GmbH & Co. KG).

Ein weiterer Aspekt der vorliegenden Erfindung ist die Bereitstellung einer Vorrichtung zum Durchführen des erfindungsgemäßen Verfahrens umfassend mindestens eine Hydriereinheit (6), einen statischen Mischer (4) und einen oder mehrere Zulaufströme (1,2), wobei der statische Mischer so konfiguriert ist, dass die Zulaufströme A und B (1,2) miteinander in Kontakt gebracht werden und anschließend über einen Strom C (5) in die Hydriereinheit (6) eingebracht werden.

Vorzugsweise umfasst die erfindungsgemäße Vorrichtung zwei, drei, vier oder mehr Zulaufströme zum statischen Mischer.

Es ist bevorzugt, dass die Hydriereinheit (6) einen oder mehrere Feststoff-Katalysatoren aufweist, vorzugsweise wobei der Feststoff-Katalysator mindestens ein Metall der achten Nebengruppe des Periodensystems der Elemente, besonders bevorzugt Ruthenium, aufweist. Es gilt das für die verwendeten Katalysatoren hierin Gesagte entsprechend.

Weiterhin ist es bevorzugt, dass in der Hydriereinheit eine Mischung aus aromatischen Verbindungen und entsprechenden alizyklischen Verbindungen, vorzugsweise eine Mischung aus aromatische Carbonsäureester und deren entsprechenden alizyklischen Verbindungen mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise aus C₈-C₁₀-Phthalat, C₈-C₁₀-Terephthalat, C₈-C₁₀-Isophthalat und C₈-C₁₀-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Di-isononylisophthalat, Di-2-ethylhexyltrimellitat und Tri-isononyltrimellitat, Diisononylphthalat und/oder Didecylphthalat und Di-isononyl-cyclohexandicarbonsäureester und/oder Di-decylcyclohexandicarbonsäureester und deren entsprechender alizyklischen Verbindungen, vorhanden ist.

Ein weiterer Aspekt der vorliegenden Erfindung ist die Verwendung eines statischen Mischers zum Inkontaktbringen von zwei oder mehr Zulaufströmen vor Einbringen in eine oder mehrere Hydriereinheiten, vorzugsweise zum Inkontaktbringen von aromatischen Verbindungen und wasserstoffhaltigem Hydriergas, vorzugsweise wobei der Mischer eine Kenics-Mischer Bauweise besitzt.

Es ist bevorzugt, dass der statische Mischer eine Geometrie aufweist, welche eine Strömung mit einer Reynoldszahl von größer als 100, vorzugsweise von größer als 200, insbesondere von größer als 500, besonders bevorzugt von größer als 900 im statischen Mischer vermittelt.

Es ist weiterhin bevorzugt, dass der statische Mischer eine Geometrie aufweist, welche eine Vermischung des in Schritt i. bereitgestellten Hydriergases und der bereitgestellten aromatischen Verbindungen in einer flüssigen Phase so vermittelt, dass der Wasserstoff aus dem wasserstoffhaltigen Hydriergas in der flüssigen Phase am Ende des statischen Mischers annähernd in Sättigungskonzentration vorliegt.

Bevorzugt im Rahmen der vorliegenden Erfindung, ist die Verwendung der erfindungsgemäß hergestellten alicyclischen Polycarbonsäureester als Weichmacher in Kunststoffen. Bevorzugte Kunststoffe sind PVC, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

Als Vertreter der obigen Gruppen seien beispielsweise folgende Kunststoffe genannt:
Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, und Isononylrest, Polymethacrylat, Polymethylmethacrylat, Methylacrylat-Butylacrylat-Copolymere, Methylmethacrylat-Butylmethacrylat-Copolymere, Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, , Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose.

Darüber hinaus können die erfindungsgemäß hergestellten alicyclischen Polycarbonsäureester zur Modifizierung von Kunststoffmischungen, beispielsweise der Mischung eines Polyolefins mit einem Polyamid, eingesetzt werden.

Neben den oben genannten Anwendungen können die erfindungsgemäß hergestellten alicyclischen Polycarbonsäureester als Schmierölkomponente, als Bestandteil von Kühlflüssigkeiten und Metallbearbeitungsflüssigkeiten verwendet werden. Ebenso können sie als Komponente in Farben, Lacken, Tinten und Klebstoffen eingesetzt werden.

## Patentansprüche

1. Verfahren zur Herstellung einer oder mehrerer alicyclischer Verbindungen, umfassend die Schritte:
i. Bereitstellen eines Stroms A aus einer oder mehrerer aromatischer Verbindungen sowie eines Stroms B aus einem wasserstoffhaltigen Hydriergas;
ii. In Kontaktbringen der in Schritt i. bereitgestellten Ströme A und B mittels mindestens eines bis maximal 8 statischen Mischern (4) unter Erhalt eines vermischten Stroms C (5) und Einbringen von Strom C (5) in eine Hydriereinheit (6);
iii. Hydrieren der einen oder mehreren aromatischen Verbindungen zu einer oder mehreren entsprechenden alicyclischen Verbindungen in der Hydriereinheit (6);
iv. Erhalten einer Produktmischung (7) umfassend eine oder mehrere alicyclische Verbindungen.

2. Verfahren gemäß Anspruch 1, wobei der statische Mischer ein Rohr umfasst, in das die beiden Ströme A und B über einen gemeinsamen Einlass zugeführt werden, wobei in dem Rohr ist mindestens ein vorzugsweise mehrere Stromelemente angeordnet sind, durch die die gewünschte Vermischung der beiden Ströme A und B erreicht wird.

3. Verfahren gemäß einem der vorangehenden Ansprüche, wobei der statische Mischer eine Bauweise, ausgewählt aus der Gruppe bestehend aus den Mischertypen Kenics-Mischer, Sulzer SMV-Mischer, Sulzer SMX-Mischer, Fluitec CSE-Mischer und Ross-ISG-Mischer, vorzugsweise eine Kenics-Mischer Bauweise, besitzt.

4. Verfahren gemäß einem der vorangehenden Ansprüche, wobei das Hydrieren in Schritt iii. an in einem Festbett der Hydriereinheit (6) angeordneten Feststoff-Katalysatoren mit dem in Schritt i. bereitgestellten wasserstoffhaltigen Hydriergas erfolgt.

5. Verfahren gemäß Anspruch 5, wobei der Katalysator mindestens ein Metall der achten Nebengruppe des Periodensystems der Elemente, vorzugweise Ruthenium, aufweist.

6. Verfahren gemäß einem der vorangehenden Ansprüche, wobei das Hydrieren in Schritt iii. bei einem Druck von 3 bis 300 bar, vorzugsweise 15 bis 200 bar, besonders bevorzugt 50 bis 150 bar, durchgeführt wird.

7. Verfahren gemäß einem der vorangehenden Ansprüche, wobei das Hydrieren in Schritt iii. bei einer Temperatur von 50°C bis 250°C, vorzugsweise 70 bis 200°C, durchgeführt wird.

8. Verfahren gemäß einem der vorangehenden Ansprüche, wobei in Schritt i. ein oder mehrere aromatische Carbonsäureester, vorzugsweise ein oder mehrere aromatische Mono-, Di- und Polycarbonsäureester, bereitgestellt werden.

9. Verfahren gemäß einem der vorangehenden Ansprüche, wobei in Schritt i. ein oder mehrere Benzol-, Diphenyl-, Naphthalin-, Diphenyloxid, Anthracen-Di- oder Polycarbonsäureester bereitgestellt werden.

10. Verfahren gemäß einem der vorangehenden Ansprüche, wobei in Schritt i. ein oder mehrere aromatische Carbonsäureester mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise C8-C10-Phthalat, C8-C10-Terephthalat, C8-C10-Isophthalat und C8-C10-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Di-isononylisophthalat, Di-2-ethylhexyltrimellitat und Tri-isononyltrimellitat, bereitgestellt werden.

11. Verfahren gemäß einem der vorangehenden Ansprüche, wobei der statische Mischer eine Geometrie aufweist, welche eine Strömung mit einer Reynoldszahl von größer als 100, vorzugsweise von größer als 200, insbesondere von größer als 500, besonders bevorzugt von größer als 900 im statischen Mischer vermittelt.

12. Vorrichtung zum Durchführen eines Verfahrens gemäß einem der Ansprüche 1 bis 11 umfassend mindestens eine Hydriereinheit (6), einen statischen Mischer (4) und einen oder mehrere Zulaufströme (1,2), wobei der statische Mischer so konfiguriert ist, dass die Zulaufströme (1,2) miteinander in Kontakt gebracht werden und anschließend über einen Strom (5) in die Hydriereinheit (6) eingebracht werden.

13. Vorrichtung gemäß Anspruch 12, wobei die Hydriereinheit (6) einen oder mehrere Feststoff-Katalysatoren aufweist, vorzugsweise wobei der Feststoff-Katalysator mindestens ein Metall der achten Nebengruppe des Periodensystems der Elemente, besonders bevorzugt Ruthenium, aufweist.

14. Vorrichtung gemäß einem der Ansprüche 12 oder 13, wobei in der Hydriereinheit eine Mischung aus aromatischen Verbindungen und entsprechenden alizyklischen Verbindungen, vorzugsweise eine Mischung aus aromatische Carbonsäureester und deren entsprechenden alizyklischen Verbindungen mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise aus C8-C10-Phthalat, C8-C10-Terephthalat, C8-C10-Isophthalat und C8-C10-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Di-isononylisophthalat, Di-2-ethylhexyltrimellitat und Tri-isononyltrimellitat, Diisononylphthalat und/oder Didecylphthalat und Di-isononyl-cyclohexandicarbonsäureester und/oder Di-decylcyclohexandicarbonsäureester und deren entsprechender alizyklischen Verbindungen, vorhanden ist.

15. Verwendung eines statischen Mischers zum Inkontaktbringen von zwei oder mehr Zulaufströmen vor Einbringen in eine oder mehrere Hydriereinheiten, vorzugsweise zum Inkontaktbringen von aromatischen Verbindungen und wasserstoffhaltigem Hydriergas, vorzugsweise wobei der Mischer eine Kenics-Mischer Bauweise besitzt.
